# EUROPEAN PATENT SPECIFICATION

(11) **EP 2 425 814 B1**
(45) Date of publication and mention of the grant of the patent: **19.06.2013**
(21) Application number: 10175337.4
(22) Date of filing: 03.09.2010
(51) Int. Cl.: A61K 9/00, A61K 9/107, A61K 47/14

(54) **A water-in-oil type emulsion for treating a disease of the eye**
Wasser-in-Öl-Emulsion zur Behandlung von Augenerkrankungen
Émulsion eau dans huile pour traiter une maladie de l'oeil

(43) Date of publication of application: 07.03.2012
(73) Proprietor: Novagali Pharma S.A., 91000 Evry (FR)
(72) Inventor: Lallemand, Frédéric, 94260 Fresnes (FR); Garrigue, Jean-Sébastien, 91370 Verrières le Buisson (FR)
(74) Representative: Icosa

(56) References cited:
- EP-A1- 1 867 323
- WO-A2-01/93911
- US-A1- 2006 121 115
- CHAN ET AL: "Phase transition water-in-oil microemulsions as ocular drug delivery systems: In vitro and in vivo evaluation", INTERNATIONAL JOURNAL OF PHARMACEUTICS, ELSEVIER BV, NL, vol. 328, no. 1, 1 December 2006 (2006-12-01), pages 65-71, XP005787883, ISSN: 0378-5173, DOI: DOI:10.1016/J.IJPHARM.2006.10.004
- TAMILVANAN S: "Oil-in-water lipid emulsions: implications for parenteral and ocular delivering systems", PROGRESS IN LIPID RESEARCH, PERGAMON PRESS, PARIS, FR, vol. 43, no. 6, 1 November 2004 (2004-11-01), pages 489-533, XP004621515, ISSN: 0163-7827, DOI: DOI:10.1016/J.PLIPRES.2004.09.001

## Description

### [Field of Invention]

The present invention relates to the field of the treatment of the conditions or diseases of the eye through the intraocular administration of therapeutic agents.

### [Background of Invention]

The treatment of eye diseases by injecting a therapeutic agent directly in the vitreous chamber has shown promising results in the past. Macugen® (oligonucleotide) and Lucentis® (monoclonal antibody) are pharmaceutical products which are efficient to treat retinal diseases.

However, their half-life in the vitreous is relatively short leading to repeated injections to maintain the effect. The rapid clearance of these products is due to the renewal of the vitreous liquid over time.

This issue was already addressed in the prior art : for example, W02009/046198 describes a method for administering a therapeutic agent in the vitreous with a sustained release kinetic; this method involves the formation of a macroscopic gel-like structure comprising said therapeutic agent, in the vitreous chamber. Also, EP2187980 describes the injection in the vitreous chamber of a therapeutic agent combined with a polymeric precursor, which will form *in situ* a hydrogel suitable for controlled release of said therapeutic agent.

However, the injection in the vitreous of a subject of a gel or gel-like structure as described in these patent applications may cause visual discomfort to the subject due to the invasion of the visual field by said gel or gel-like structure.

In another approach, a solid implant is injected in the eye of the subject, and the implant will release the active ingredient over several months. However, this form of administration may not be suitable for proteins and monoclonal antibodies.

Therefore, there remains a need for a method for sustaineously releasing in the vitreous chamber, a composition comprising a hydrophilic therapeutic agent, such as for example a protein or a nucleic acid. Ensuring the visual comfort of the patient when the composition is within the vitreous body is another issue.

Surprisingly, the Applicant realized that a water-in-oil emulsion could be an efficient vehicle for administering hydrophilic therapeutic agents. Water-in-oil type emulsions are biphasic systems in which water droplets are dispersed within an oil phase.

The use of water-in-oil type emulsions as vehicles for sustained release of therapeutic agents is well known in the art. For example, WO01/89479 discloses the use of water-in-oil type emulsions for the parenteral administration of hydrophilic active ingredients with a sustained release kinetic. Chan et al. (Int. J. Pharm. 2007 Jan 2;328(1):65-71) specifically studied the use of water-in-oil type emulsions for topical delivery of an ocular drug with a sustained release kinetic. However, these prior art documents do not suggest the use of water-in-oil type emulsions for intraocular administration of a drug with a sustained release kinetic.

This invention thus relates to the use of water-in-oil type emulsions for intraocular administration of a therapeutic agent to a subject in need thereof, providing a sustained release kinetic, and avoiding any invasion of the field of vision of the subject or safety issues.

An advantage of the solution proposed by the Applicant is that the water-in-oil emulsion of the invention forms a bubble having a lower density than the vitreous liquid ; when injected, the bubble of composition will slowly shift up from injection location to the upper part of the vitreous. Consequently, this liquid bubble will float over the vitreous, out of the visual field, avoiding any visual discomfort for the subject to which the composition is administered. Moreover, the composition of the invention being in physical contact with both vitreous body and targeted tissues such as, for example, the choroid or the retina, the release of the therapeutic agent will occur at the exact location of need.

### [Definitions]

In the present invention, the following terms have the following meanings:
- "Emulsion": colloidal system made of two non-miscible elements, for example oil and water. One element (the dispersed phase) is present on the form of droplets dispersed in the other element, constituting the continuous phase.
- "Water-in-oil type emulsion": emulsion made of water or aqueous droplets (i.e. the dispersed phase) dispersed in an oil phase (i.e. the continuous phase). A water-in-oil type emulsion also comprises surfactants (as defined hereafter), to avoid phase separation.
- "Sustained release kinetic": describes the slow release kinetic of a compound, at a predetermined rate and over an extended period of time.
- "Intraocular administration": injection of a product directly in the eyeball i.e. injection in the anterior chamber or in the posterior cavity (vitreous cavity) of the eye.
- "Surfactant": defines a substance that lowers the interfacial tension between two liquids.
- "Bioresorbable": defines a compound that progressively disappears in a biologic environment.
- "Therapeutic agent": describes a molecule or a substance, preferably a biological molecule such as for example an oligonucleotide, a siRNA, a miRNA, a DNA fragment, an aptamer, an antibody and the like, or a chemical entity, having the capacity, when administered in a suitable amount, of slowing down or stopping the progression, aggravation, or deterioration of one or more symptoms of a disease, or condition; alleviates the symptoms of a disease or condition; cures a disease or condition.
- "Therapeutically effective amount": the amount of a therapeutic agent necessary and sufficient for slowing down or stopping the progression, aggravation, or deterioration of one or more symptoms of the disease, or condition; alleviating the symptoms of the disease or condition ; curing the disease or condition.
- "Hydrophilic": defines a molecule or a portion of a molecule that is typically charge-polarized and capable of hydrogen bonding, enabling it to dissolve more readily in water than in oil or other solvents.
- "Lipophilic": refers to a chemical compound capable to dissolve in fats, oils, lipids, and nonpolar solvents.
- "Non-miscible": liquid which does not combine or blend with another liquid, or which does not combine or blend immediately with another liquid.

### [Summary]

This invention relates to composition for administering with a sustained release kinetic a therapeutically effective amount of a therapeutic agent to a subject in need thereof for treating diseases or conditions of the eye, wherein the composition is a water-in-oil type emulsion comprising an oil phase, a lipophilic surfactant dissolved in the oil phase, an aqueous phase dispersed in the oil phase, a hydrophilic therapeutic agent dissolved in the aqueous dispersed phase, wherein the composition is intraocularly injected, and wherein the composition has a density lower than 1.

According to an embodiment, the oil phase is selected from the group comprising triglycerides such as, for example, medium chain or long chain triglycerides, monoglycerides, diglycerides, vegetable oils or mineral oils.

Preferably, the lipophilic surfactant is selected from the group comprising sorbitan ester such as, for example, sorbitan stearate, sorbitan laurate and sorbitan monopalmitate, bentonite, glycerol monostearate and propylene glycol monolaurate or mixtures thereof.

In a preferred embodiment, the aqueous phase is present in the composition of the invention in an amount ranging from 0.1 to less than 50% in weight to the total weight of the composition, preferably from 0.5 to 15% w/w, more preferably from 2 to 10% w/w. Preferably, the hydrophilic therapeutic agent is selected from the group comprising monoclonal antibodies (full or fragment Fab), such as for example ranibizumab; anti-angiogenic or anti-complement molecules, such as for example anginex or lodamin; a ROCK (Rho-kinases) inhibitor, such as for example fasudil; tetrapyridoether against dry age macular degeneration; small peptides such as for example anti-B1 peptide R-954 to proteins such as anti-CD160 S-HLA-G; enzymes such as for example superoxide dismutase or catalase; WNT3A protein which activates WNT (Wingless - Integration site) for survival of photoreceptor cells; growth factors such as epithelium growth factors (EGF), anti-EGF or TGF (Transforming growth factor); siRNA such as siRNA anti-arginase, miRNA; oligonucleotides such as antisens DNA or antisens RNA; antioxidant small molecules such as EUK (Eukaryon) family, for example EUK-143 sodium catalase mimetic; iron chelating molecules such as deferiprone and salicylaldehyde isonicotinoyl hydrazone; anti-inflammatory molecules such as epigallocatechin gallate; free radical scavengers such as edaravone; or antibiotics for back of the eye infection such as linezolide, anti-inflammatory molecules preferably selected from the group comprising lipophilic cyclosporine A, dexamethasone and its hydrophilic derivatives, or mixtures thereof. In one embodiment of the invention, the composition further comprising a lipophilic therapeutic agent in the oil phase, said lipophilic therapeutic agent being selected from the group comprising lutein, alpha-tocopherol and dexamethasone-palmitate. According to the invention, the composition may further comprise viscosity modifying agents, such as, for example an hydrogel, and/or pH buffering agents, such as, for example, phosphate, citrate, tris, histidine or acetate buffer, and/or osmolality modifying agents, such as, for example NaCl, KCl, CaCl₂, glycerol, mannitol, alpha-trehalose or propylene glycol. In a preferred embodiment, the composition of the invention is intravitreally injectable. The diseases or conditions of the eye that may be treated with the composition of the invention are selected from the group comprising glaucoma, anterior uveitis retinal oxidation, age related macular degeneration, posterior uveitis, diabetic macular edema and central vein occlusion. This invention also relates to a pharmaceutical composition comprising the water-in-oil type emulsion of the invention, and further comprising one or more pharmaceutically acceptable excipients. This invention also relates to a medicament comprising a water-in-oil type emulsion as described above. This invention also relates to a device comprising the composition or the medicament according to the invention. According to a preferred embodiment, the composition, the pharmaceutical composition, the medicament or the device are not implants.

This invention also relates to a composition for use in the treatment by intraocular injection of diseases or condition of the eye according to claims 1-9, wherein a volume of 5 to 250 microliters of the composition is injected in the vitreous chamber or in the anterior chamber. According to the invention, the injected composition forms *in situ* a bubble within which the aqueous phase migrates towards the surface of a bubble, for sustained release of the therapeutic agent to the vitreous chamber, to the anterior chamber or the targeted tissue.

### [Detailed Description]

The invention thus relates to a composition for administering with a sustained release kinetic a therapeutically effective amount of a therapeutic agent to a subject in need thereof for treating diseases or conditions of the eye, wherein the composition is a water-in-oil type emulsion comprising an oil phase, a lipophilic surfactant dissolved in the oil phase, an aqueous phase dispersed in the oil phase and a hydrophilic therapeutic agent dissolved in the aqueous dispersed phase, the composition being intraocularly injected and having a density lower than 1.

Due to the fact that its continuous phase is essentially composed of oil, the water-in-oil type emulsion of the invention presents a lower density than the vitreous liquid which has a density equivalent, if not equal, to the density of water. Thus, according to the invention, the density of the water-in-oil type emulsion of the invention is less than 1. Preferably, the density of the water-in-oil type emulsion ranges from 0.9 to 0.99, more preferably from 0.94 to 0.98. Therefore, when injected, the solution will form a liquid, non breakable and non miscible bubble. When injected in the vitreous body, the bubble will be located over the vitreous liquid.

In one embodiment of the invention, the emulsion further comprises one or more lipophilic surfactants, in an amount sufficient for ensuring the water-in-oil type of the emulsion. In a particular embodiment of the invention, said lipophilic surfactants are selected from the group comprising sorbitan ester such as, for example, sorbitan stearate, sorbitan laurate, and sorbitan monopalmitate, bentonite, glycerol monostearate and propylene glycol monolaurate or mixtures thereof.

In a particular embodiment of the invention, the HLB (hydrophilic - lipophilic Balance) of the surfactants of the composition ranges from 0 to 9, preferably from 2 to 8.

In a particular embodiment of the invention, the amount of lipophilic surfactants in the water-in-oil type emulsion ranges from 0.1 to 10% in weight to the weight of the total emulsion, preferably from 0.5 to 5% w/w, more preferably from 1 to 2% w/w.

In one embodiment of the invention, the aqueous phase in the water-in-oil type emulsion is present in an amount ranging from 0.1 to less than 50% in weight to the weight of the total emulsion, preferably from 0.5 to 15% w/w, more preferably from 2 to 10% w/w. Preferably, said aqueous phase is water or is essentially composed water.

In a particular embodiment of the invention, the composition includes one or more hydrophilic therapeutic agent(s) present in the aqueous droplets of the water-in-oil type emulsion.

In one embodiment of the invention, the hydrophilic therapeutic agent is selected from the group comprising monoclonal antibodies (full or fragment Fab), such as for example ranibizumab; anti-angiogenic or anti-complement molecules, such as for example anginex or lodamin; a ROCK (Rho-kinases) inhibitor, such as for example fasudil; tetrapyridoether against dry age macular degeneration; small peptides such as for example anti-B1 peptide R-954 to proteins such as anti-CD160 S-HLA-G; enzymes such as for example superoxide dismutase or catalase; WNT3A protein which activates WNT (Wingless - Integration site) for survival of photoreceptor cells; growth factors such as epithelium growth factors (EGF), anti-EGF or TGF (Transforming growth factor); siRNA such as siRNA anti-arginase, miRNA; oligonucleotides such as antisens DNA or antisens RNA; antioxidant small molecules such as EUK (Eukaryon) family, for example EUK-143 sodium catalase mimetic; iron chelating molecules such as deferiprone and salicylaldehyde isonicotinoyl hydrazone; anti-inflammatory molecules such as epigallocatechin gallate; free radical scavengers such as edaravone; or antibiotics for back of the eye infection such as linezolide, anti-inflammatory molecules preferably selected from the group comprising lipophilic cyclosporine A, dexamethasone and its hydrophilic derivatives and mixtures thereof.

In an embodiment of the invention, the amount of hydrophilic therapeutic ingredient in the emulsion ranges from 0.01 to 10% in weight to the total weight of the emulsion, preferably from 0.05 to 5% w/w, more preferably from 0.1 to 1% w/w.

In an embodiment of the invention, the emulsion further comprises one or more lipophilic therapeutic agents in the oil phase. In a preferred embodiment of the invention, said lipophilic therapeutic agent is selected from the group comprising lutein, alpha-tocopherol and dexamethasone-palmitate.

In a preferred embodiment, the amount of hydrophilic therapeutic ingredient in the emulsion ranges from 0.01 to 10% in weight to the total weight of the emulsion, preferably from 0.05 to 5% w/w, more preferably from 1 to 2% w/w.

The water-in-oil type emulsion of the invention is efficient for sustained release administration of a therapeutic agent. Said sustained release effect is provided by the migration of water droplets dispersed in the continuous oil phase to the surface of the oil bubble formed by the emulsion when injected in the eye. In one embodiment of the invention, the sustained release kinetic can be adapted to the exact need of the patient.

In a first embodiment of the invention, said sustained release kinetic may depend on the physic-chemical properties of the oil phase. The more viscous the oil phase is, the more extended the period of release may be. With viscous oil such as long chain triglycerides, the release may be extended up to one year. In one embodiment of the invention, the oil phase of the water-in-oil type emulsion comprises an oil selected from the group comprising triglycerides such as, for example semi-synthetic oils : medium chain triglycerides (MCT) or long chain triglycerides; monoglycerides, diglycerides or vegetable oils such as, for example, castor oil ormineral oils. According to an embodiment, the viscosity of the oil phase ranges from 1 to 10000 mPa.s at 20°C, preferably from 10 to 5000 mPa.s at 20°C, even more preferably from 25 to 1000 mPa.s at 20°C.

In a second embodiment of the invention, said sustained release kinetic may depend on the size of the water droplets dispersed in the oil phase. The smaller the droplets are, the longer their migration to the surface of the injected bubble may be, and then the more extended the period of release may be. For example, for comparable compositions of the invention in terms of ingredients, an emulsion with a droplet size of more than 1 µm may release the therapeutic agent in about 1 week to 2 months, whereas the release may be increased to more than 2 months when the droplet size is below 500 nm.

In a third embodiment of the invention, said sustained release kinetic may be conditioned by the volume of the injected water-in-oil type emulsion. The bigger the emulsion bubble is, the more extended the period of release may be. Preferably, a volume of the composition of the invention ranging from 5 to 250 µL, preferably from 10 to 100 µL, more preferably about 50 µL is injected.

In a fourth embodiment of the invention, the viscosity of the aqueous phase is increased in order to enhance the sustained release. In a particular embodiment of the invention, said viscosity is increased by addition of a hydrogel. In a preferred embodiment of the invention, said hydrogel is made of cellulose, hyaluronic acid, and/or collagen.

In a fifth embodiment of the invention, the means for sustaineously releasing the therapeutic agents as described in the first to four embodiments hereabove, may be combined one to each other or all together in order to modulate the sustain release effect.

According to an embodiment of the invention, the aqueous phase of the emulsion further comprises a pH modifying agent or a pH buffering agent. In a preferred embodiment, said pH buffering agent is selected from the group comprising phosphate, citrate, tris, histidine or acetate buffers. In a preferred embodiment, said pH buffering agent is a phosphate buffer. In one embodiment of the invention, the amount of said agent for modifying the pH of the aqueous phase ranges from 0.05 to 10% in weight to the total weight of the aqueous phase, preferably from 0.01 to 5% w/w, more preferably from 0.1 to 1% w/w.

According to an embodiment of the invention, the aqueous phase of the emulsion further comprises an agent for modifying the osmolality of the aqueous phase of the emulsion. In a first embodiment, said agent for modifying the osmolality is selected from the group comprising NaCl, KCl and CaCl₂. In a second embodiment, the modification of the osmolality of the composition results from the addition of a compound selected from the group comprising neutral compounds such as, but not limited to, glycerol, mannitol, alpha-trehalose or propylene glycol. In a preferred embodiment, the modification of the osmolality of the composition results from the addition of 0.5-2%, preferably 0.9% w/w of NaCl, 0.5-10%, preferably 3-5% w/w of alpha-trehalose or mannitol or propylene glycol in weight to the weight of the total emulsion.

In one embodiment of the invention, if the water-in-oil type emulsion is too viscous to be injected, the emulsion can be re-emulsified into a water phase to form a multiple emulsion of the type water-in-oil-in-water.

According to an embodiment, the composition is intraocularly injectable. Preferably, the composition is intravitreally injectable.

The water-in-oil type emulsion according to the invention is bioresorbable. In one embodiment of the invention, the oily bubble is resorbed in a period of time ranging from 1 to 24 months after injection, preferably from 6 to 18 months after injection, more preferably about 12 months after injection.

The water-in-oil type emulsion according to the invention is for use in treating diseases or conditions of the eye. In one embodiment of the invention, said diseases or conditions of the eye are selected from the group comprising glaucoma, anterior uveitis retinal oxidation, age related macular degeneration, posterior uveitis, diabetic macular edema and central vein occlusion.

The present invention also relates to a pharmaceutical composition according to the water-in-oil type emulsion of the invention. In one embodiment of the invention, the pharmaceutical composition further comprises at least one pharmaceutically acceptable excipient.

The present invention also relates to a medicament according to the water-in-oil type emulsion of the invention.

The present invention also relates to a device for administering the water-in-oil type emulsion, the pharmaceutical composition or the medicament according to the invention. Preferably, said device is a prefilled syringe. In one embodiment of the invention, said device contains the pharmaceutical composition or the medicament according to the invention.

Also, the present invention relates to a composition for use in the treatment by intraocular injection of a condition or disease of the eye, comprising administering intraocularly a therapeutic amount of the composition or of the medicament of the invention. Preferably, the invention comprises the injection, preferably in the vitreous chamber, of a volume ranging from 5 to 250 µL, preferably from 10 to 100 µL, more preferably of about 50 µL. In a preferred embodiment, said composition or medicament is injected less than once a week, preferably less than once a month, more preferably less than once in six months. According to an embodiment, the injected composition forms *in situ* a bubble within which the aqueous phase migrates towards the surface of a bubble, letting the therapeutic agent be sustaineously released to the vitreous chamber or the targeted tissue.

The invention is further illustrated by the following examples.

### [Examples]

### Example 1: composition comprising ranibizumab

| **Ingredients** | **Concentration** |
|---|---|
| ranibizumab | 0.1% |
| Water for injection | 4% |
| dihydrated alpha, alpha-trehalose | 3% |
| Monohydrated histidine chlorhydrate histidine | 0.05% |
| Sorbitan stearate | 2% |
| Medium chain triglyceride | Qs 100% |

### Example 2: composition comprising R-954

| **Ingredients** | **Concentration** |
|---|---|
| Peptide R-954 | 0.8% |
| Water for injection | 5% |
| Glycerol monostearate | 0.5% |
| Sorbitan monoplamitate | 1% |
| Medium chain triglyceride | Qs 100% |
| Dexamethasone palmitate | 1.2% |

## Claims

1. A composition for use in the treatment by intraocular injection of diseases or conditions of the eye, wherein the composition is a water-in-oil type emulsion comprising an oil phase, a lipophilic surfactant dissolved in the oil phase, an aqueous phase dispersed in the oil phase, a hydrophilic therapeutic agent dissolved in the aqueous dispersed phase, and wherein the composition has a density lower than 1.

2. The composition for use according to claim **1**, wherein the oil phase is selected from the group comprising triglycerides such as, for example, medium chain or long chain triglycerides, monoglycerides, diglycelides, vegetable oils or mineral oils.

3. The composition for use according to anyone of claim **1** or claim **2,** wherein the lipophilic surfactant is selected from the group comprising sorbitan ester such as, for example, sorbitan stearate, sorbitan laurate and sorbitan monopalmitate, bentonite, glycerol monostearate and propylene glycol monolaurate or mixtures thereof.

4. The composition for use according to anyone of claims **1** to **3,** wherein the aqueous phase is present in an amount ranging from 0.1 to less than 50% in weight to the total weight of the composition, preferably from 0.5 to 15% w/w, more preferably from 2 to 10% w/w.

5. The composition for use according to anyone of claims **1** to **4,** wherein said hydrophilic therapeutic agent is selected from the group comprising monoclonal antibodies (full or fragment Fab), such as for example ranibizumab; anti-angiogenic or anti-complement molecules, such as for example anginex or lodamin; a ROCK (Rho-kinases) inhibitor, such as for example fasudil; tetrapyridoether against dry age macular degeneration; anti-B1 peptide R-954 to proteins such as anti-CD160 S-HLA-G; enzymes such as for example superoxide dismutase or catalase; WNT3A protein which activates WNT (Wingless - Integration site) for survival of photoreceptor cells; growth factors such as epithelium growth factors (EGF), anti-EGF or TGF (Transforming growth factor); siRNA such as siRNA anti-arginase, miRNA; oligonucleotides such as antisens DNA or antisens RNA; antioxidant small molecules such as EUK (Eukaryon) family, for example EUK-143 sodium catalase mimetic; iron chelating molecules such as deferiprone and salicylaldehyde isonicotinoyl hydrazone; anti-inflammatory molecules such as epigallocatechin gallate; free radical scavengers such as edaravone; or antibiotics for back of the eye infection such as linezolide, anti-inflammatory molecules preferably selected from the group comprising lipophilic cyclosporine A, dexamethasone and its hydrophilic derivatives, or mixtures thereof.

6. The composition for use according to anyone of claims **1** to **5,** further comprising a lipophilic therapeutic agent in the oil phase, said lipophilic therapeutic agent being selected from the group comprising lutein, alpha-tocopherol and dexamethasone-palmitate.

7. The composition for use according to anyone of claims **1** to **6**, further comprising viscosity modifying agents, such as, for example an hydrogel, and/or pH buffering agents, such as, for example, phosphate, citrate, tris, histidine or acetate buffer, and/or osmolality modifying agents, such as, for example NaCl, KCI, CaCl₂, glycerol, mannitol, alpha-trehalose or propylene glycol.

8. The composition for use according to anyone of claims **1** to **7**, for use in the treatment by intravitreal injection of diseases or conditions of the eye.

9. The composition for use according to anyone of claims **1** to **8**, wherein said diseases or conditions of the eye to be treated arc selected from the group comprising glaucoma, anterior uveitis retinal oxidation, age related macular degeneration, posterior uveitis, diabetic macular edema and central vein occlusion.

10. The composition for use in the treatment by intraocular injection of diseases or conditions of the eye according to anyone of claims **1** to **9**, which is a
pharmaceutical composition comprising one or more pharmaceutically acceptable excipients.

11. The composition for use in the treatment by intraocular injection of diseases or conditions of the eye according to anyone of claims **1** to **9**, which is a medicament.

12. The composition for use in the treatment by intraocular injection of diseases or conditions of the eye according to anyone of claims **1** to **9**, which is comprised in a device.

13. The composition for use in the treatment by intraocular injection of diseases or conditions of the eye according to anyone of claims **1** to **9,** wherein a volume of 5 to 250 microliters of the composition is injected in the vitreous chamber or in the anterior chamber.

14. The composition for use in the treatment by intraocular injection of diseases or conditions of the eye according to anyone of claims **1** to **9**, wherein the injected composition has sustained release kinetic due to the *in situ* formation of a bubble within which the aqueous phase migrates towards the surface of a bubble, for sustained release of the therapeutic agent to the vitreous chamber, to the anterior chamber or the targeted tissue.

## Patentansprüche

1. Zusammensetzung zur Verwendung bei der Behandlung von Erkrankungen oder Zuständen des Auges durch intraokulare Injektion, wobei die Zusammensetzung eine Emulsion vom Typ Wasser-in-Öl ist, umfassend eine Ölphase, ein lipophiles Tensid, das in der Ölphase gelöst ist, eine wässrige Phase, die in der Ölphase dispergiert ist, ein hydrophiles therapeutisches Mittel, das in der wässrigen dispergierten Phase gelöst ist, und wobei die Zusammensetzung eine Dichte von weniger als 1 aufweist.

2. Zusammensetzung zur Verwendung nach Anspruch **1**, wobei die Ölphase ausgewählt ist aus der Gruppe bestehend aus Triglyceriden wie z.B. mittelkettigen oder langkettigen Triglyceriden, Monoglyceriden, Diglyceriden, pflanzlichen Ölen oder Mineralölen.

3. Zusammensetzung zur Verwendung nach einem von Anspruch **1** oder Anspruch **2**, wobei das lipophile Tensid ausgewählt ist aus der Gruppe bestehend aus Sorbitanester wie z.B. Sorbitanstearat, Sorbitanlaurat und Sorbitanmonopalmitat, Bentonit, Glycerolmonostearat und Propylenglykolmonorautat oder Mischungen davon.

4. Zusammensetzung zur Verwendung nach einem der Ansprüche **1** bis **3**, wobei die wässrige Phase in einer Menge vorhanden ist, die von 0,1 bis weniger als 50 Gew.% zum Gesamtgewicht der Zusammensetzung reicht, vorzugsweise von 0,5 bis 15 Gew.% , insbesondere von 2 bis 10 Gew.%.

5. Zusammensetzung zur Verwendung nach einem der Ansprüche **1** bis **4**, wobei das hydrophile therapeutische Mittel ausgewählt ist aus der Gruppe bestehend aus monoklonalen Antikörpern (Voll- oder Fragment-Fab) wie z.B. Ranibizumab; antiangiogenen oder antikomplementären Molekülen wie z.B. Anginex oder Lodamin; einen ROCK (Rho-Kinasen)-Inhibitor wie z.B. Fasudil; Tetrapyridoether gegen altersbedingte trockene Makuladegeneration; anti-B 1-Peptid R-954 für Proteine wie z.B. Anti-CD160 S-HLA-G; Enzymen wie z.B. Superoxiddismutase oder -Katalase; WNT3A-Protein, das WNT (Wingless-Integrationsstelle) zum Überleben von Photorezeptorzellen aktiviert; Wachstumsfaktoren wie z.B. Epithelwachstumsfaktoren (EGF), Anti-EGF oder TGF (Transformationswachstumsfaktor); siRNA wie z.B. siRNA Anti-Arginase, miRNA; Oligonukleotide wie z.B. Antisens-DNA oder Antisens-RNA; kleine Antioxidans-Moleküle wie z.B. die Gruppe der EUK (Eukaryon), z.B. EUK-143 Natriumkatalase-Mimetikum; Eisenchelatierungsmoleküle wie z.B. Deferipron und Salicylaldehydisonicotinoylhydrazon, entzündungshemmende Moleküle wie z.B. Epigallocatechingallat; Freie-Radikale-Abfangmittel wie z.B. Edavaron; oder Antibiotika für Infektionen der hinteren Augenabschnitte wie z.B. Linezolid, entzündungshemmende Moleküle, vorzugsweise ausgewählt aus der Gruppe bestehend aus lipohilem Cyclosporin A, Dexamethason und seinen hydrophilen Derivaten, oder Mischungen davon.

6. Zusammensetzung zur Verwendung nach einem der Ansprüche **1** bis **5**, weiter umfassend ein lipophiles therapeutisches Mittel in der Ölphase, wobei das lipophile therapeutische Mittel ausgewählt ist aus der Gruppe bestehend aus Lutein, Alpha-Tocopherol und Dexamethasonpalmitat.

7. Zusammensetzung zur Verwendung nach einem der Ansprüche **1** bis **6**, weiter umfassend viskositätsmodifizierende Mittel wie z.B. ein Hydrogel, und/oder pH-Puffermittel wie z.B. Phosphat, Citrat, Tris, Histidin oder Acetatpuffer, und/oder osmolalitätsmodifizierende Mittel wie z.B. NaCl, KCl, CaCl₂, Glycerol, Mannitol, alpha-Trehalose oder Propylenglykol.

8. Zusammensetzung zur Verwendung nach einem der Ansprüche **1** bis **7** zur Verwendung bei der Behandlung von Erkrankungen oder Zuständen des Auges mit intravitrealer Injektion.

9. Zusammensetzung zur Verwendung nach einem der Ansprüche **1** bis **8**, wobei die Erkrankungen oder Zustände des Auges, die behandelt werden sollen, ausgewählt sind aus der Gruppe bestehend aus Glaukom, anteriore Uveitis, Netzhautoxidation, altersbedingte Makuladegeneration, posteriore Uveitis, diabetisches Makulaödem und zentraler Venenverschluss.

10. Zusammensetzung zur Verwendung bei der Behandlung durch intraokulare Injektion von Erkrankungen oder Zuständen des Auges nach einem der Ansprüche **1** bis **9**, wobei es sich um eine pharmazeutische Zusammensetzung umfassend eines oder mehrerer pharmazeutisch annehmbaren Trägerstoffe handelt.

11. Zusammensetzung zur Verwendung bei der Behandlung durch intraokulare Injektion von Erkrankungen oder Zuständen des Auges nach einem der Ansprüche **1** bis **9**, wobei es sich um ein Arzneimittel handelt.

12. Zusammensetzung zur Verwendung bei der Behandlung durch intraokulare Injektion von Erkrankungen oder Zuständen des Auges nach einem der Ansprüche **1** bis **9,** die in einer Vorrichtung enthalten ist.

13. Zusammensetzung zur Verwendung bei der Behandlung durch intraokulare Injektion von Erkrankungen oder Zuständen des Auges nach einem der Ansprüche **1** bis **9**, wobei ein Volumen von 5 bis 250 Mikroliter der Zusammensetzung in die Glaskörperkammer oder in die Augenvorderkammer injiziert wird.

14. Zusammensetzung zur Verwendung bei der Behandlung durch intraokulare Injektion von Erkrankungen oder Zuständen des Auges nach einem der Ansprüche **1** bis **9**, wobei die injizierte Zusammensetzung eine verzögerte Freisetzungskinetik hat, aufgrund der *in situ*-Bildung einer Blase umfasst, in der die wässrige Phase an die Oberfläche einer Blase migriert, um das therapeutische Mittel an die Glaskörperkammer, an die Augenvorderkammer oder an das Zielgewebe verzögert abzugeben.

## Revendications

1. Composition pour son utilisation dans le traitement par injection intraoculaire de maladies ou de conditions de l'oeil, dans laquelle la composition est une émulsion de type eau-dans-huile comprenant une phase huileuse, un surfactant lipophile dissous dans la phase huileuse, une phase aqueuse dispersée dans la phase huileuse, un agent thérapeutique hydrophile dissous dans la phase aqueuse dispersée, et dans laquelle la composition a une densité inférieure à 1.

2. La composition pour son utilisation selon la revendication **1**, dans laquelle la phase huileuse est sélectionnée dans le groupe comprenant les triglycérides tels que par exemple les triglycérides à chaîne moyenne ou longue, les monoglycérides, les diglycérides, les huiles végétales ou les huiles minérales.

3. La composition pour son utilisation selon la revendication **1** ou la revendication **2**, dans laquelle le surfactant lipophile est sélectionné dans le groupe comprenant les esters de sorbitan tels que par exemple le stéarate de sorbitan, le laurate de sorbitan et le monopalmitate de sorbitan, la bentonite, le monostéarate de glycérol, le monolaurate de propylène glycol ou leurs mélanges.

4. La composition pour son utilisation selon l'une quelconque des revendications **1** à **3**, dans laquelle la phase aqueuse est présente dans une quantité allant de 0,1 à moins de 50% en poids par rapport au poids total de la composition, de préférence de 0,5 à 15% p/p, plus préférentiellement de 2 à 10% p/p.

5. La composition pour son utilisation selon l'une quelconque des revendications **1** à **4**, dans laquelle ledit agent thérapeutique hydrophile est sélectionné dans le groupe comprenant les anticorps monoclonaux (entiers or fragments Fab), tels que par exemple le ranibizumab; les molécules anti-angiogéniques ou anti-compléments, telles que par exemple l'anginex ou la lodamine; un inhibiteur de ROCK (Rho-kinases), tel que par exemple le fasudil; le tétrapyridoéther contre la dégénérescence maculaire sèche liée à l'âge; le peptide R-954 anti-B1 aux protéines, telles que anti-CD160 S-HLA-G; les enzymes telles que par exemple la superoxyde dismutase ou la catalase; la protéine WNT3A qui active le WNT (site d'intégration Wingless) pour la survie des cellules photoréceptrices; les facteurs de croissance tels que les facteurs de croissance de l'épithélium (EGF), anti-EGF ou TGF (facteur de croissance transformant); les siARN tel que le siARN anti-arginase, les miARN; les oligonucléotides tels que l'ADN antisens ou l'ARN antisens; les petites molécules antioxydantes telles que la famille EUK (Eukaryon), par exemple le mimétique EUK-143 sodium catalase; les molécules chélatant le fer telles que la défériprone et l'hydrazone d'isonicotinoyle salicylaldéhyde; les molécules anti-inflammatoires telles que le gallate d'épigallocatéchine; les pièges de radicaux libres tels que l'édaravone; ou les antibiotiques pour les infections de l'arrière de l'oeil tels que le linézolide, les molécules anti-inflammatoires, sélectionnées de préférence dans le groupe comprenant la cyclosporine A lipophile, la dexaméthasone et ses dérivés hydrophiles, ou leurs mélanges.

6. La composition pour son utilisation selon l'une quelconque des revendications **1** à **5**, comprenant en outre un agent thérapeutique lipophile dans la phase huileuse, ledit agent thérapeutique lipophile étant sélectionné dans le groupe comprenant la lutéine, l'alpha-tocophérol et la dexaméthasone palmitate.

7. La composition pour son utilisation selon l'une quelconque des revendications **1** à **6**, comprenant en outre des agents de modification de la viscosité, tels que par exemple un hydrogel, et/ou des agents tampon de pH, tels que par exemple un tampon phosphate, citrate, tris, histidine ou acétate, et/ou des agents de modification de l'osmolalité, tels que par exemple NaCl, KCl, CaCl₂, glycérol, mannitol, alpha-tréhalose ou propylène glycol.

8. La composition pour son utilisation selon l'une quelconque des revendications **1** à **7,** pour son utilisation dans le traitement par injection intravitréenne de maladies ou de conditions de l'oeil.

9. La composition pour son utilisation selon l'une quelconque des revendications **1** à **8**, dans laquelle lesdites maladies ou conditions de l'oeil à traiter sont sélectionnées dans le groupe comprenant le glaucome, l'uvéite antérieure, l'oxydation de la rétine, la dégénérescence maculaire liée à l'âge, l'uvéite postérieure, l'oedème maculaire diabétique et l'occlusion de la veine centrale.

10. La composition pour son utilisation dans le traitement par injection intraoculaire de maladies ou de conditions de l'oeil selon l'une quelconque des revendications **1** à **9**, qui est une composition pharmaceutique comprenant un ou plus excipients pharmaceutiquement acceptables.

11. La composition pour son utilisation dans le traitement par injection intraoculaire de maladies ou de conditions de l'oeil selon l'une quelconque des revendications **1** à **9**, qui est un médicament.

12. La composition pour son utilisation dans le traitement par injection intraoculaire de maladies ou de conditions de l'oeil selon l'une quelconque des revendications **1** à **9**, qui est comprise dans un dispositif.

13. La composition pour son utilisation dans le traitement par injection intraoculaire de maladies ou de conditions de l'oeil selon l'une quelconque des revendications **1** à **9**, dans laquelle un volume de 5 à 250 microlitres de la composition est injectée dans la chambre vitrée ou dans la chambre antérieure.

14. La composition pour son utilisation dans le traitement par injection intraoculaire de maladies ou de conditions de l'oeil selon l'une quelconque des revendications **1** à **9**, dans laquelle la composition injectée a une cinétique de libération prolongée due à la formation *in situ* d'une bulle dans laquelle la phase aqueuse migre vers la surface de la bulle, pour la libération prolongée de l'agent thérapeutique dans la chambre vitrée, dans la chambre antérieure ou dans le tissu ciblé.
